(19) Europäisches Patentamt
European Patent Office
Office européen des brevets

(11) Veröffentlichungsnummer: **0 059 356**
**B1**

# (12) EUROPÄISCHE PATENTSCHRIFT

(45) Veröffentlichungstag der Patentschrift: **02.05.85**

(21) Anmeldenummer: **82101091.5**

(22) Anmeldetag: **15.02.82**

(51) Int. Cl.⁴: **C 07 D 271/04**, C 07 D 413/04, A 61 K 31/495, A 61 K 31/41, A 61 K 31/38

(54) **Substituierte 3-Amino-sydnonimine, Verfahren zu ihrer Herstellung und ihre Verwendung.**

(30) Priorität: **02.03.81 DE 3107933**

(43) Veröffentlichungstag der Anmeldung: **08.09.82 Patentblatt 82/36**

(45) Bekanntmachung des Hinweises auf die Patenterteilung: **02.05.85 Patentblatt 85/18**

(84) Benannte Vertragsstaaten: **AT BE CH DE FR GB IT LI NL SE**

(56) Entgegenhaltungen:
**EP - A - 0 023 343**
**DE - A - 2 241 991**
**US - A - 3 833 589**

(73) Patentinhaber: **CASSELLA Aktiengesellschaft, Hanauer Landstrasse 526, D-6000 Frankfurt am Main 61 (DE)**

(72) Erfinder: **Schönafinger, Karl, Dr., Parkstrasse 1, D-8531 Uehlfeld (DE)**
Erfinder: **Beyerle, Rudi, Dr., An der Pfaffenmauer 44, D-6000 Frankfurt am Main 60 (DE)**
Erfinder: **Bohn, Helmut, Dr., Kranzbergring 11, D-6369 Schöneck (DE)**
Erfinder: **Just, Melitta, Dr., Hüttenberg 6, D-6369 Schöneck 1 (DE)**
Erfinder: **Martorana, Piero A., Dr., Kaiser-Friedrich-Promenade 108, D-6380 Bad Homburg v.d.H. (DE)**
Erfinder: **Nitz, Rolf-Eberhard, Dr., Heinrich-Bingemer-Weg 64, D-6000 Frankfurt am Main 60 (DE)**

(74) Vertreter: **Urbach, Hans-Georg, Dr. et al, Hanauer Landstrasse 526, D-6000 Frankfurt am Main 61 (DE)**

**EP 0 059 356 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft pharmakologisch wirksame substituierte 3-Amino-sydnonimine der allgemeinen Formel I

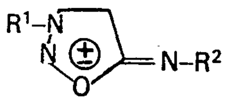

(I)

und ihre pharmakologisch annehmbaren Säureadditionssalze, worin

$R^1$ den Rest

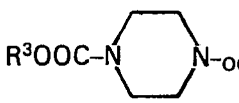

oder den Rest

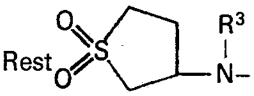

$R^2$ Wasserstoff oder den Rest $-CO-R^4$,

$R^3$ Methyl, Ethyl, Propyl, Isopropyl,

$R^4$ einen aliphatischen Rest mit 1 bis 4 C-Atomen, der auch durch Alkoxi mit 1 bis 3 C-Atomen substiutiert sein kann, einen cycloaliphatischen Rest mit 5 bis 7 C-Atomen, einen bicycloaliphatischen Rest mit 7 bis 14 C-Atomen, einen tricycloaliphatischen Rest mit 7 bis 16 C-Atomen, einen Alkoxirest mit 1 bis 6 C-Atomen, einen Aryloxirest mit 6 bis 12 C-Atomen oder einen Alkoxicarbonylrest mit insgesamt 2 bis 7 C-Atomen, einen Arylrest mit 6 bis 12 C-Atomen, einen durch 1 bis 3 Halogenatome und/oder 1 bis 3 Alkylreste mit 1 bis 3 C-Atomen und/oder 1 bis 3 Alkoxireste mit 1 bis 3 C-Atomen und/oder 1 oder 2 Nitrogruppen mono-, di- oder trisubstituierten Arylrest mit 6 bis 12 C-Atomen, bedeutet.

Die Erfindung betrifft ferner ein Verfahren zur Herstellung der erfindungsgemässen Verbindungen.

Die für $R^4$ stehenden aliphatischen Reste, Alkoxireste oder Alkoxicarbonylreste können geradkettig oder verzweigt sein. Als für $R^4$ stehende aliphatische Reste kommen insbesondere Alkylreste mit 1 bis 4 C-Atomen in Betracht. Als für $R^4$ stehende aliphatische Reste, die durch Alkoxi mit 1 bis 3 C-Atomen substituiert sind, ist insbesondere der Methoxi-methyl-rest zu nennen. Als für $R^4$ stehende cycloaliphatische Reste kommen vor allem Cycloalkylreste mit 5 bis 7 C-Atomen, insbesondere Cyclopentyl, und bevorzugt Cyclohexyl in Betracht. Als für $R^4$ stehender bicycloaliphatischer Rest kommt insbesondere das 2.6.6-Trimethylbicyclo [3.1.1]-heptan -3yl(=Pinanyl) in Betracht. Als für $R^4$ stehender tricycloaliphatischer Rest kommt insbesondere das Tricyclo[3.3.1.1$^{3,7}$]-decan-1-yl(=Adamantanyl) in Betracht. Als für $R^4$ stehende Alkoxireste kommen insbesondere Methoxi- und Ethoxi-reste in Betracht. Als für $R^4$ stehender Alkoxicarbonylrest kommt insbesondere der Ethoxicarbonyl-rest in Betracht. Als für $R^4$ stehende Arylreste sind z.B. α- oder β-Naphthylreste, insbesondere aber der Phenylrest, zu nennen. Als für $R^4$ stehende Aryloxireste sind z.B. α- oder β-Naphtoxi-reste, insbesondere aber der Phenoxi-rest, zu nennen. Die für $R^4$ stehenden Arylreste können mono-, di- oder trisubstituiert sein, wobei jedoch auch bei einer Trisubstitution nur maximal 2 Nitrogruppen vorhanden sein können, wie beispielsweise 2-Methyl-4,6-dinitrophenyl und 2-Chlor-6-methyl -4-nitrophenyl. Als Halogensubstituenten für die Arylreste kommen z.B. Chlor und Bromatome in Betracht. Als für $R^4$ stehende substituierte Arylreste sind insbesondere zu nennen: Methyl-phenyl-(Tolyl), Nitro-phenyl und Chlor-phenyl.

Für $R^3$ sind Ethyl und Methyl bevorzugt. Für $R^4$ sind bevorzugt: Methyl, Ethyl, Cyclohexyl, Phenyl, 4-Chlor-phenyl und 4-Nitro-phenyl. Für $R^1$ ist der Rest 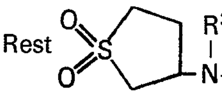, insbesondere mit $R^3$-Methyl, sowie insbesondere in Kombination mit $R^2$-Wasserstoff bevorzugt.

Die Verbindungen der allgemeinen Formel I können dadurch hergestellt werden, dass Verbindungen der allgemeinen Formel II

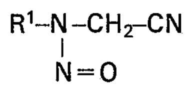

(II)

zu Verbindungen der allgemeinen Formel Ia

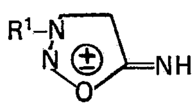

(Ia)

cyclisiert und für den Fall, dass $R^2 = -COR^4$ ist, die Verbindungen der Formel Ia oder ihre Säureadditionssalze mit Acylierungsmitteln, die den Rest $-COR^4$ einführen, acyliert und die so erhaltenen Verbindungen gegebenenfalls in Säureadditionssalze überführt werden.

Die Cyclisierung der Verbindungen II zu den Verbindungen Ia wird in einem geeigneten organischen oder anorganischen Lösungsmittel, beispielsweise Wasser, einem Alkanol mit 1 bis 4 C-Atomen, einem Carbonsäurealkylester, beispielsweise Essigsäureethylester, oder einem Gemisch derartiger Lösungsmittel, wie Wasser-Methanol oder vorzugsweise Essigsäureethylester-Methanol, unter Zusatz eines Cyclisierungsmittels, normalerweise bei Temperaturen von 0 bis 40 °C, vorzugsweise bei 0 bis 20 °C, durchgeführt. Als Cyclisierungsmittel sind solche geeignet, die in wässriger Lösung einen pH-Wert unter 3 einstellen, also z.B. Mineralsäuren, wie Schwefel-, Salpeter- oder Phosphorsäure, vorzugsweise Chlorwasserstoff, aber auch starke organische Säuren, wie Trifluoressigsäure. Bei der Cyclisierung wird das entsprechende Säureadditionssalz der Verbindung Ia erhalten. Die Verbindungen der Formel Ia stellen erfindungsgemässe Verbindungen für den Fall dar, dass $R^2$ = Wasserstoff ist.

Die Acylierung der Verbindungen der Formel Ia zur Einführung des Restes $R^2 = -COR^4$ kann in an sich bekannter Weise mit geeigneten Acylierungsmitteln der Formel III

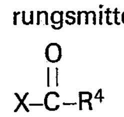

(III)

worin X z.B. Halogen, insbesondere Chlor, $-O-\overset{\displaystyle O}{\overset{\|}{C}}-R^4$, Aryloxi, insbesondere Tolyloxi, Dinitrophenyloxi oder Nitrophenyloxi bedeutet, durchgeführt werden. Die Acylierung wird in einem geeigneten Lösungsmittel, wie z.B. Wasser, oder einem polaren organischen Lösungsmittel, wie Dimethylformamid, Dimethylsulfoxid, Pyridin, einem Lösungsmittelgemisch, wie z.B. Wasser/Methylenchlorid oder in einem Überschuss des Acylierungsmittels, zweckmässigerweise unter Rühren, bei Temperaturen von 0 °C bis zur Siedetemperatur des Lösungs- oder Acylierungsmittels, vorzugsweise von 0 bis 20 °C, durchgeführt. Bei der Acylierung ist die Anwesenheit eines säurebindenden Mittels, wie z.B. Pyridin, Natriumhydrogencarbonat oder Natriumacetat, zweckmässig.

Die substituierten 3-Amino-sydnonimine der allgemeinen Formel I bilden mit anorganischen oder organischen Säuren Säureadditionssalze. Zur Bildung derartiger Säureadditionssalze sind anorganische und organische Säuren geeignet. Geeignete Säuren sind beispielsweise Chlorwasserstoff, Bromwasserstoff, Naphthalindisulfonsäuren, insbesondere Naphthalindisulfonsäure(1,5), Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze können wie üblich durch Vereinigung der Komponenten, zweckmässigerweise in einem geeigneten Lösungs- oder Verdünnungsmittel, hergestellt werden. Bei der Synthese der Verbindungen der Formel Ia fallen die Säureadditionssalze an. Aus den Säureadditionssalzen können die freien Verbindungen der allgemeinen Formel I bzw. Ia gewünschtenfalls in bekannter Weise, d.h. durch Auflösen oder Suspendieren in Wasser und Alkalischstellen, z.B. mit Natronlauge, und anschliessendes Isolieren, gewonnen werden.

Die benötigten Ausgangsverbindungen der allgemeinen Formel II können in an sich bekannter Weise nach der Strecker'schen Aminonitrilsynthese aus Verbindungen der allgemeinen Formel IV

$$R^1-NH_2 \qquad (IV)$$

durch Umsetzung mit Formaldehyd und Blausäure bzw. Natriumcyanid in einem geeigneten Lösungsmittel, z.B. Wasser, hergestellt werden, wobei zunächst eine Verbindung der allgemeinen Formel V

$$R^1-NH-CH_2-CN \qquad (V)$$

entsteht, die durch Nitrosierung in die Verbindung II überführt wird. Die Nitrosierung wird in bekannter Weise in einem geeigneten Lösungsmittel, vorzugsweise in Wasser, bei Temperaturen von 0 bis 10 °C durchgeführt. Die salpetrige Säure wird dabei normalerweise aus einem Alkalimetallnitrit und Salzsäure erzeugt. Es ist zweckmässig, die wässrige Lösung der Verbindung V mit Salzsäure auf einen pH-Wert von 1 bis 3 einzustellen und das Alkalimetallnitrit in Form einer wässrigen Lösung zu der gerührten und abgekühlten Lösung der Verbindung zuzutropfen.

Die Lösung der erhaltenen Verbindung II kann direkt der Cyclisierungsreaktion unterworfen werden. Normalerweise ist es jedoch angebracht, die Nitrosoverbindung II zunächst in einem geeigneten organischen Lösungsmittel aufzunehmen und in ihm, gegebenenfalls nach Zusatz eines weiteren Lösungsmittels, die Cyclisierung zu der Verbindung der Formel I durchzuführen.

Die Verbindungen der Formel IV sind zum Teil bekannt bzw. lassen sich nach den folgenden Umsetzungen herstellen:

$$\begin{array}{lll} R^1-H & + KNCO & \rightarrow R^1-CO-NH_2 \\ V & \quad VI & \quad VII \end{array}$$

$$\begin{array}{lll} R^1-CO-NH_2 & + NaOCl & \rightarrow R^1-NH_2 \\ VII & \quad VIII & \quad IV \end{array}$$

Dabei werden Verbindungen V zunächst in bekannter Weise mit Kaliumcyanat VI zu den Verbindungen VII umgesetzt, die wiederum in bekannter Weise durch Oxidation mit Natriumhypochlorit nach dem Hoffmann-Abbau in die Verbindungen IV überführt werden. Bei der angegebenen Bedeutung von $R^1$ sind die Verbindungen V als Amine, die Verbindungen VII als Harnstoffe und die Verbindungen IV als Hydrazine zu bezeichnen.

Die Verbindungen der allgemeinen Formel I und ihre pharmakologisch annehmbaren Säureadditionssalze besitzen wertvolle pharmakologische Eigenschaften.

Besonders ausgeprägt ist ihre Wirkung auf das Herz-Kreislauf-System. Verglichen mit der im Handel befindlichen strukturähnlichen Verbindung Molsidomin wirken sie in niedrigeren Dosen und über einen noch längeren Zeitraum. Sie senken beispielsweise den Blutdruck ebenso wie den Pulmonalarteriendruck und den linksventrikulären enddiastolischen Druck und tragen so zu einer Entlastung der Herztätigkeit im Sinne einer antianginösen Wirkung bei, ohne dabei eine reflektorische Tachykardie zu provozieren. Gegenüber den ähnlich gebauten Verbindungen der DE-OS 29 30 736 besitzen sie eine geringere Toxizität.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon, neben üblichen pharmazeutisch einwandfreien Träger- und Zusatzstoffen enthalten.

Die Heilmittel können oral, z.B. in Form von Pillen, Tabletten, Lacktabletten, Dragees, Hart- und Weichgelatinekapseln, Lösungen, Sirupen, Emulsionen oder Suspensionen oder Aerosolmischungen verabreicht werden. Die Verabreichung kann aber auch rektal, z.B. in Form von Suppositorien, oder parenteral, z.B. in Form von Injektionslösungen, oder perkutan, z.B. in Form von Salben oder Tinkturen, erfolgen.

Zur Herstellung der pharmazeutischen Präparate können pharmazeutisch inerte anorganische oder organische Trägerstoffe verwendet werden. Für die Herstellung von Pillen, Tabletten, Dragees und Hartgelatinekapseln kann man z.B. Lactose, Maisstärke oder Derivate davon, Talk, Stearinsäure oder deren Salze etc. verwenden. Trägerstoffe für Weichgelatinekapseln und Suppositorien sind z.B. Fette, Wachse, halbfeste und flüssige Polyole, natürliche oder gehärtete Öle etc. Als Trägerstoffe für die Herstellung von Lösungen und Sirupen eignen sich z.B. Wasser, Saccharose, Invertzucker, Glukose, Polyole etc. Als Trägerstoffe für die Herstellung von Injektionslösungen eignen sich z.B. Wasser, Alkohole, Glyzerin, Polyole oder pflanzliche Öle.

Die pharmazeutischen Präparate können neben den Wirk- und Trägerstoffen noch Zusatzstoffe, wie z.B. Füllstoffe, Streck-, Spreng-, Binde-, Gleit-, Netz-, Stabilisierungs-, Emulgier-, Konservierungs-, Süss-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblokker, wie z.B. Propranolol, Pindolol, Metoprolol; Vasodilatatoren, wie z.B. Carbochromen; Beruhigungsmittel, wie z.B. Barbitursäurederivate, 1,4-Benzodiazepine und Meprobamat; Diuretica, wie z.B. Chlorothiazid; das Herz tonisierende Mittel, wie z.B. Digitalispräparate; blutdrucksenkende Mittel, wie z.B. Hydralazin, Dihydralazin, Prazosin, Clonidin, Rauwolfia-Alkaloide; Mittel, die den Fettsäurespiegel im Blut senken, wie z.B. Bezafibrat, Fenofibrat; Mittel für die Thromboseprophylaxe, wie z.B. Phenprocoumon.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoffe enthalten, können am Menschen bei der Bekämpfung bzw. Vorbeugung von Erkrankungen des kardiovaskulären Systems verwendet werden, beispielsweise als antihypertensive Heilmittel bei den verschiedenen Formen des Bluthochdrucks, bei der Bekämpfung bzw. Vorbeugung von Angina pectoris usw. Die Dosierung kann innerhalb weiter Grenzen variieren und ist in jedem einzelnen Fall den individuellen Gegebenheiten anzupassen. Im allgemeinen ist bei oraler Verabreichung pro menschlichem Individuum eine Tagesdosis von etwa 0,5 bis 100 mg, vorzugsweise 1 bis 20 mg, angemessen. Auch bei anderen Applikationsformen liegt die Tagesdosis, wegen der guten Resorption der Wirkstoffe, in ähnlichen Mengenbereichen, d.h. im allgemeinen ebenfalls bei 0,5 bis 100 mg/Mensch. Die Tagesdosis wird normalerweise in mehrere, z.B. 2 bis 4 Teilverabreichungen aufgeteilt.

Zum Nachweis der antianginösen Wirkung der erfindungsgemässen Verbindungen wurden Untersuchungen an Bastardhunden beiderlei Geschlechts in Pentobarbital-Narkose (30 bis 40 mg/kg i.v.) oder in Urethan-Chloralose-Narkose (3 ml/kg Urethan-Chloralose-Gemisch i.v. = 20 mg/kg Chloralose und 250 mg/kg Urethan) durchgeführt. Die Beatmung der Tiere erfolgte mit einem Bird-Mark -7-Respirator. Der endexpiratorische Kohlensäuregehalt (gemessen mit einem Ultrarotabsorptionsschreiber) betrug zwischen 4,5 und 5 Vol.-%. Während des gesamten Versuchs erhielten die Tiere mit Pentobarbital-Narkose eine Dauerinfusion von Pentobarbital i.v. = 4 mg (in 6 ml)/kg/h um eine konstante Narkosetiefe zu gewährleisten. Die Tiere mit Urethan-Chloralose-Narkose erhielten keine Dauerinfusion. Die Infusion wurde durch die Vena cephalica gegeben. Nach der Präparation des Versuchstieres wurde ca. 1 Stunde gewartet, bis sich alle haemodynamischen Parameter eingestellt hatten (steady state). Danach wurde mit dem eigentlichen Versuch begonnen.

Zur Bestimmung des mittleren peripheren Blutdrucks (= BD) wurden der systolische und diastolische Blutdruck peripher in der Arteria femoralis über einen Statham-Druckaufnehmer gemessen. Ein über die Arteria carotis in den linken Ventrikel geschobener Millar-Tip-Katheter lieferte das Signal für den linksventrikulären enddiastolischen Druck (= LVEDP) und die Herzfrequenz (= HF). Mit einem zweiten, über die Vena jugularis eingeschobenen Tip-Katheter wurde der mittlere Blutdruck (= PAP) in der Arteria pulmonalis erfasst.

Die erhaltenen Ergebnisse sind in der nachfolgenden Tabelle angegeben:

| Substanz | Dosis mg/kg | LVEDP Δ mm Hg | PAP Δ mm Hg | BD Δ mm Hg | HF Δ b/min |
|---|---|---|---|---|---|
| A | 0,05 | −2 | −1 | −40 | 0 |
| B | 0,1 | −5 | −5 | −60 | +15 |
| C | 0,01 | −1 | −5 | −25 | +10 |
| D | 0,1 | −4 | −4,5 | −70 | +25 |
| E | 0,01 | −2,5 | −2 | −20 | +10 |
| F | 0,1 | −3 | −6 | −70 | −5 |
| MOL | 0,1 | −2,3 | −1,6 | −18 | +3 |
| ISDN | 0,1 | −3,5 | −2,1 | − 6 | + 7 |

In der vorstehenden Tabelle bedeuten:

A = 3- (N-Methyl -N- (tetrahydro -3-thienyl-S,S-dioxid) -amino) -sydnonimin-hydrochlorid

B = 3- (4-Ethoxicarbonylpiperazin -1-yl) -sydnonimin-hydrochlorid

C = 3- (N-Methyl-N- (tetrahydro -3-thienyl-S,S-dioxid) -amino) -$N^6$-(4-nitrobenzoyl) -sydnonimin

D = 3- (N-Methyl-N- (tetrahydro -3-thienyl-S,S-dioxid) amino) -$N^{6'}$ cyclohexylcarbonyl -sydnonimin-hydrochlorid

E = 3- (4- Ethoxicarbonylpiperazin -1-yl) -$N^6$- cyclohexyl-carbonyl-sydnonimin

F = 3- (4- Ethoxicarbonylipiperazin -1-yl)-$N^6$acetylsydnonimin

MOL = Molsidomin (Vergleichssubstanz)

ISDN = Isosorbiddinitrat (Vergleichssubstanz)

LVEDP = Linksventrikulärer enddiastolischer Druck

PAP = Mittlerer Pulmonalarteriendruck

BD = Mittlerer peripherer Blutdruck

HF = Herzfrequenz (Δb/min = Schläge pro Minute)

Die EP-A-23 343 betrifft Sydnonimin-Derivate, die in 3-Stellung durch Thiomorpholino, Tetrahydro-1,4-thiazin-4- yl-1-oxid oder 4-Methansulfonyl-piperazin-1-yl substituiert sind. Einige dieser Verbindungen zeigen im Tierversuch an der Maus in hohen Dosen zentralnervöse Nebenwirkungen. Derartige Nebenwirkungen sind bei den Verbindungen der vorliegenden Erfindung auch in hohen Dosen nicht zu beobachten.

Die DE-A-17 70 061 betrifft 3-Amino-4-bromsydnonimine, deren Aminogruppe durch Alkyl- oder Aralkylgruppen substituiert ist oder mit diesen Gruppen ein heterocyclisches Ringsystem bildet. Gegenüber diesen bekannten Verbindungen sind die erfindungsgemässen Verbindungen pharmakologisch wirksamer.

In den folgenden Beispielen sind Prozentangaben Gewichtsprozente. Zers. bedeutet Zersetzung.

Beispiel 1

3- (N-Methyl-N- (tetrahydro -3- thienyl-S,S-dioxid) -amino -sydnonimin-hydrochlorid.

20,5 g 1-Methyl-1- (tetrahydro -3- thienyl-S,S-dioxid) -hydrazin-hydrochlorid werden in 120 ml Wasser gelöst. Dazu wird bei 0 bis 5 °C eine Lösung von 4,9 g Natriumcyanid in 10 ml Wasser und anschliessend ebenfalls bei 0 bis 5 °C 8,3 ml einer 40%igen Formalinlösung getropft. Dann lässt man das Reaktionsgemisch auf Raumtemperatur erwärmen und rührt 15 h nach, kühlt dann auf 0 bis 5 °C ab und stellt mit ca. 8 ml konz. Salzsäure einen pH-Wert von 1 bis 2 ein. 6,9 g Natriumnitrit werden in 15 ml Wasser gelöst und bei 0 bis 5 °C zugetropft wobei sich ein Öl abscheidet. Dieses wird mit Essigsäureethylester (100 ml) ausgeschüttelt, die organische Phase über Natriumsulfat getrocknet. Nach Zugabe von 100 ml Methanol werden bei 5 bis 10 °C innerhalb von 2 bis 3 Stunden insgesamt etwa 70 bis 80 g Chlorwasserstoff eingeleitet. Nun wird auf 0 °C abgekühlt und 2 Stunden nachgerührt, abgesaugt und aus einem Isopropanol/Wasser-Gemisch umkristallisiert: Fp=177 bis 179 °C, Ausbeute: 8,3 g (35% der Theorie).

Die Cyclisierung verläuft ähnlich, wenn man anstelle von Chlorwasserstoff Schwefel-, Salpeter-, Phosphor- oder Trifluoressigsäure verwendet und/oder die Cyclisierung bei Temperaturen von 0 bis 40 °C durchführt und/oder das Methanol durch eine entsprechende Menge Essigsäureethylester, Ethanol, i-Propanol, n-Propanol, i-Butanol oder n-Butanol ersetzt.

Beispiel 2

3- (4-Ethoxicarbonylpiperazin -1yl)- sydnonimin-hydrochlorid. 21 g 1-Ethoxicarbonyl -4-aminopiperazin-hydrochlorid werden in 120 ml Wasser gelöst. Dann wird bei einer Temperatur von 0 bis 5 °C eine Lösung von 4,9 g Natriumcyanid in 10 ml Wasser und anschliessend ebenfalls bei 0 bis 5 °C 8,3 ml einer 40%igen Formalinlösung zugetropft. Dann lässt man die Mischung sich auf Raumtemperatur erwärmen und rührt 15 Stunden nach, kühlt auf 0 bis 5 °C ab und stellt mit ca. 8 ml konz. Salzsäure einen pH-Wert von 1 bis 2 ein. 6,9 g Natriumnitrit werden in 15 ml Wasser gelöst und bei 0 bis 5 °C zugetropft, wobei sich ein Öl abscheidet. Dieses wird mit Essigsäureethylester (100 ml) ausgeschüttelt, die organische Phase über Natriumsulfat getrocknet. Nach Zusatz von 100 ml Methanol werden bei 5 bis 10 °C innerhalb von 2 bis 3 Stunden insgesamt etwa 70 bis 80 g Chlorwasserstoff eingeleitet. Nun wird auf 0 °C abgekühlt und 2 Stunden nachgerührt, abgesaugt und aus Isopropanol umkristallisiert: Fp=170 bis 171 °C, Ausbeute: 12,6 g (45% der Theorie).

Die Cyclisierung verläuft ähnlich, wenn man anstelle von Chlorwasserstoff Schwefel-, Salpeter-, Phosphor- oder Trifluoressigsäure verwendet und/oder die Cyclisierung bei Temperaturen von 0 bis 40 °C durchführt und/oder das Methanol durch eine entsprechende Menge Essigsäureethylester, Ethanol, i-Propanol, n-Propanol, i-Butanol oder n-Butanol ersetzt.

Beispiel 3

3-(N-Methyl-N- (tetrahydro -3thienyl-S,S-dioxid) -amino)-$N^6$- ethoxicarbonyl-sydnonimin.

5,4 g 3-(N-Methyl-N- (tetrahydro -3- thienyl-S,S-dioxid) -amino) -sydnonimin-hydrochlorid und 4,2 g Natriumhydrogencarbonat werden in 50 ml Wasser gelöst und mit der Lösung von 3,25 g Chlorameisensäureethylester in 50 ml Methylenchlorid vereinigt.

Nach 24 stündigem Rühren bei Raumtemperatur wird abgesaugt, die Methylenchloridphase eingeengt, der Rückstand mit dem abgesaugten Feststoff vereinigt und aus 30 ml Methanol umkristallisiert: Fp=139 bis 142 °C, Ausbeute: 2,3 g (38% der Theorie.

Analog diesem Beispiel lassen sich die folgenden Verbindungen synthetisieren, wobei hinter dem Schmelzpunkt angegeben ist, in welchem Lösungsmittel und bei welcher Reaktionstemperatur die Acylierung durchgeführt wird:

3- (N-Methyl-N- (tetrahydro -3- thienyl-S,S-dioxid) -amino) -$N^6$-benzoyl-sydnonimin Fp= 152 bis 153 °C, in Wasser/Methylenchlorid bei 10 °C,

3- (N-Methyl-N- (tetrahydro -3- thienyl-S,S-dioxid) -amino) -$N^6$- (4-nitrobenzoyl) -sydnonimin Fp=221 bis 222 °C (Zers.), in Wasser/Methylenchlorid bei 20 °C,

3- (N-Methyl-N- (tetrahydro -3- thienyl-S,S-dioxid) -amino) -$N^6$-cyclohexylcarbonyl-sydnonimin-hydrochlorid Fp=150 °C (Zers.) in Wasser bei 0 °C,

3- (N-Methyl-N- (tetrahydro -3- thienyl-S,S-dioxid) -amino) -$N^6$- (4-methylbenzoyl) -sydnonimin Fp=146 bis 149 °C, in Wasser/Methylenchlorid bei 20 °C,

3- (4-Ethoxicarbonylpiperazin -1- yl)-$N^6$- ethoxicarbonylsydnonimin, Fp=170 bis 172 °C, in Wasser bei 20 °C,

3- (4-Ethoxicarbonylpiperazin -1- yl)-$N^6$-cyclohexylcarbonyl -sydnonimin Fp=136 bis 137 °C, in Wasser bei 0 °C,

3- (4-Ethoxicarbonylpiperazin -1-yl)-$N^6$benzoyl-sydnonimin Fp=159 bis 160 °C, in Wasser/ Methylenchlorid bei 25 °C,

3-(4-Ethoxicarbonylpiperazin -1-yl)- $N^6$-(ethoxicarbonylcarbonyl) -sydnonimin Fp=123 bis 124 °C, in Wasser/Methylenchlorid bei 0 °C,

3- (4-Ethoxicarbonylpiperazin -1-yl) -$N^6$- (4-chlorbenzoyl) -sydnonimin Fp=203 bis 207 °C (Zers.), in Wasser/Methylenchlorid bei 20 °C,

3- (4-Ethoxicarbonylpiperazin -1- yl)-$N^6$- pivaloyl-sydnonimin Fp=151 bis 152 °C, in Wasser bei 10 °C,

3- (4-Ethoxicarbonylpiperazin -1-yl)- $N^6$- (1-adamantylcarbonyl) -sydnonimin Fp=215 bis 216 °C, in Wasser/Methylenchlorid bei 20 °C

3- (4-Ethoxicarbonylpiperazin -1-yl) -$N^6$- (3-[−]pinanylcarbonyl) -sydnonimin Fp=145 bis 146 °C, in Wasser/Methylenchlorid bei 20 °C,

3- (N-Methyl-N- (tetrahydro -3- thienyl-S,S-dioxid) -amino)- $N^6$- (4-methoxibenzoyl)- sydnonimin Fp=140 bis 143 °C, in Dimethyl-formamid bei 10 °C,

3- (N-Ethyl-N- (tetrahydro -3- thienyl-S,S-dioxid) -amino) -$N^6$- methoxicarbonyl-sydnonimin Fp 140–143 °C, in Wasser bei 0 °C,

3- (N-Methyl-N- (tetrahydro -3- thienyl-S,S-dioxid) -amino) -$N^6$- (4-chlorbenzoyl) -sydnonimin Fp 141–143 °C, in Wasser/Methylenchlorid bei 20 °C,

3- (N-Methyl-N- (tetrahydro- 3- thienyl-S,S-dioxid) -amino) -$N^6$- pivaloyl-sydnonimin Fp 160–162 °C, in Wasser bei 10 °C,

3-N- (Methyl-N- (tetrahydro -3- thienyl-S,S-dioxid) -amino)$N^6$- ethoxicarbonyl- carbonyl-sydnonimin Fp 147–150 °C, in Wasser/Methylenchlorid bei 0 °C,

3- (4-Ethoxicarbonylpiperazin -1- yl)-$N^6$-methoxicarbonyl-sydnonimin Fp 181–183 °C, in Wasser bei 0 °C,

3- (4-Ethoxicarbonylpiperazin -1- yl)-$N^6$- (4-methylbenzoyl)-sydnonimin Fp 165–166 °C, in Wasser/Methylenchlorid bei 20 °C,

3- (4-Ethoxicarbonylpiperazin -1-yl)-$N^6$- (4-nitrobenzoyl) -sydnonimin Fp 210–212 °C, in Wasser/Methylenchlorid bei 20 °C.

Beispiel 4

3- (4-Ethoxicarbonyl-piperazin -1-yl)-$N^6$-acetyl-sydnonimin. 5,6 g 3-(4-Ethoxicarbonylpiperazin -1- yl)-sydnonimin-hydrochlorid werden in einer Mischung aus 20 ml Acetanhydrid und 20 ml abs. Pyridin 14 Stunden bei Raumtemperatur gerührt. Der Niederschlag wird abgesaugt und mit Methylenchlorid nachgewaschen, Fp=164 bis 165 °C; Ausbeute 3,5 g (62% der Theorie).

Analog diesem Beispiel lassen sich die folgenden Verbindungen synthetisieren, wobei hinter dem Schmelzpunkt angegeben ist, mit welchem Acylierungsmittel und bei welcher Reaktionstemperatur die Acylierung durchgeführt wird:

3- (N-Methyl-N- (tetrahydro -3- thienyl-S,S-dioxid) -amino-$N^6$methoxiacetyl-sydnonimin, Fp 151–153 °C, in Methoxiacetanhydrid/Pyridin bei 20 °C,

3- (N-Ethyl-N- (tetrahydro -3- thienyl-S,S-dioxid) -amino)-$N^6$- acetyl-sydnonimin, Fp 162–164 °C in Acetanhydrid bei 5 °C,

3- (4-Methoxicarbonyl-piperazin -1-yl)-$N^6$-acetyl-sydnonimin, Fp 188–191 °C, in Acetanhydrid bei 50 °C,

3- (4-Methoxicarbonyl-piperazin -1-yl)-$N^6$-methoxiacetyl-sydnonimin, Fp 148–151 °C in Methoxiacetanhydrid/Pyridin bei 40 °C.

In den nachfolgenden Beispielen werden pharmazeutische Präparate beschrieben.

Beispiel 5

Gelatineweichkapseln, enthaltend 5 mg Wirkstoff pro Kapsel:

| | |
|---|---|
| | pro Kapsel |
| 3- (N-Methyl-N- (tetrahydro -3- thienyl-S,S- dioxid)- amino)-sydnonimin | 5 mg |
| Aus Kokosfett fraktioniertes Triglyceridgemisch | 150 mg |
| Kapselinhalt | 155 mg |

Beispiel 6

Injektionslösung, enthaltend 1 mg Wirkstoff pro ml:

| | |
|---|---|
| | pro ml |
| 3- (N-Methyl-N- (tetrahydro -3- thienyl-S,S- dioxid)-amino) -sydnonimin-hydrochlorid | 1,0 mg |
| Polyäthylenglycol 400 | 0,3 ml |
| Natriumchlorid | 2,7 mg |
| Wasser zu Injektionszwecken | ad 1 ml |

Beispiel 7

Emulsionen, enthaltend 3 mg Wirkstoff pro 5 ml

| | |
|---|---|
| | pro 100 ml Emulsion |
| 3- (4-Ethoxicarbonylpiperazin-1-yl)-sydnonimin-hydrochlorid | 0,06 g |
| Neutralöl | q.s. |
| Natriumcarboximethylcellulose | 0,6 g |
| Polyoxiäthylen-stearat | q.s. |
| Glycerin rein | 0,2 bis 2,0 g |
| Geschmacksstoff | q.s. |
| Wasser (entsalzt oder destilliert) | ad 100 ml |

Beispiel 8

Rektale Arzneiform, enthaltend 4 mg Wirkstoff pro Suppositorium

| | |
|---|---|
| | pro Suppositorium |
| 3- (4-Ethoxicarbonylpiperazin-1-yl)-sydnonimin | 4 mg |
| Suppositoriengrundmasse | ad 2 g |

Beispiel 9

Tabletten, enthaltend 2 mg Wirkstoff pro Tablette

| | |
|---|---|
| | pro Tablette |
| 3-(N-Methyl-N (tetrahydro-3-thienyl-S,S-dioxid)-amino)-$N^6$-benzoyl-sydnonimin-Lactat (feingemahlen) | 2 mg |
| Maisstärke (weiss) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboximethylstärke | 25 mg |
| | 309 mg |

**Patentansprüche: für die Vertragsstaaten BE, CH, DE, FR, GB, IT, LI, NL, SE**

1. Substituierte 3-Amino-sydnonimine der allgemeinen Formel I

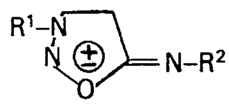

(I)

und ihre pharmakologisch annehmbaren Säureadditionssalze, worin

$R^1$ den Rest

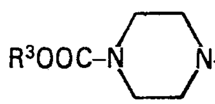

oder den Rest

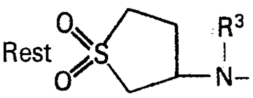

$R^2$ Wasserstoff oder den Rest $-CO-R^4$,

$R^3$ Methyl, Ethyl, Propyl, Isopropyl,

$R^4$ einen aliphatischen Rest mit 1 bis 4 C-Atomen, der auch durch Alkoxi mit 1 bis 3 C-Atomen substituiert sein kann, einen cycloaliphatischen Rest mit 5 bis 7 C-Atomen, einen bicycloaliphatischen Rest mit 7 bis 14 C-Atomen, einen tricycloaliphatischen Rest mit 7 bis 16 C-Atomen, einen Alkoxirest mit 1 bis 6 C-Atomen, einen Aryloxirest mit 6 bis 12 C-Atomen, oder einen Alkoxicarbonylrest mit insgesamt 2 bis 7 C-Atomen, einen Arylrest mit 6 bis 12 C-Atomen, einen durch 1 bis 3 Halogenatome und/oder 1 bis 3 Alkylreste mit 1 bis 3 C-Atomen und/oder 1 bis 3 Alkoxireste mit 1 gbis 3 C-Atomen und/oder 1 oder 2 Nitrogruppen mono-, di- oder trisubstituierten Arylrest mit 6 bis 12 C-Atomen bedeutet.

2. Substituierte 3-Amino-sydnonimine nach Anspruch 1, dadurch gekennzeichnet, dass

$R^3$ Methyl oder Ethyl

$R^4$ mit 1 bis 4 C-Atomen, Methoxi-methyl, Cycloalkyl mit 5 bis 7 C-Atomen, 2.6.6-Trimethyl-bicyclo [3.1.1]-heptanyl, Tricyclo-[3.3.1.$1^{3,7}$]-decanyl, Methoxi, Ethoxi, Phenoxi, Ethoxicarbonyl, Phenyl, Methylphenyl, Nitrophenyl, Chlorphenyl bedeuten.

3. Substituierte 3-Amino-sydnonimine nach Anspruch 1 oder 2, dadurch gekennzeichnet, dass $R^1$ die N-Methyl-N- (tetrahydro -3- thienyl-S,S-dioxid)- amino-Gruppe bedeutet.

4. Substituierte 3-Amino-sydnonimine nach den Ansprüchen 1 bis 3, dadurch gekennzeichnet, dass $R^2$ Wasserstoff oder die Gruppe $-COR^4$ bedeutet und $R^4$ Methyl, Ethyl, Cyclohexyl, Phenyl, 4-Chlorphenyl oder 4-Nitrophenyl bedeutet.

5. 3-(N-Methyl-N- (tetrahydro -3- thienyl-S,S-dioxid) -amino)-sydnonimin oder ein Säureadditionssalz davon.

6. 3-(4-Ethoxicarbonylpiperazin-1-yl)-sydnonimin oder ein Säureadditionssalz davon.

7. 3-(N-Methyl-N- (tetrahydro -3- thienyl-S,S-dioxid) -amino)$N^6$- nitrobenzoyl-sydnonimin oder ein Säureadditionssalz davon.

8. Verfahren zur Herstellung der Verbindungen der Ansprüche 1 bis 7, dadurch gekennzeichnet, dass eine Verbindung der allgemeinen Formel II

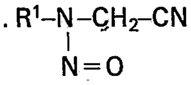

(II)

zu einer Verbindung der allgemeinen Formel Ia, die auch in Form eines Säureadditionssalzes vorliegen kann

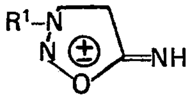

(Ia)

cyclisiert und gegebenenfalls aus dem Säureadditionssalz die freie Verbindung der Formel Ia isoliert und sie oder ein Säureadditionssalz davon mit Acylierungsmitteln, die den Rest $-COR^4$ einführen, acyliert und die erhaltene Verbindung gegebenenfalls in ein Säureadditionssalz überführt wird.

9. Verfahren nach Anspruch 8, dadurch gekennzeichnet, dass die Cyclierung in einem Lösungs- oder Dispergiermittel bei Temperaturen von 0 bis 40 °C, vorzugsweise 0 bis 20 °C, mit Hilfe von Cyclisierungsmitteln, die in wässriger Lösung einen pH-Wert unter 3 einstellen, durchgeführt wird.

10. 3-Amino-sydnonimine, der Ansprüche 1 bis 7 als pharmakologische Wirkstoffe, zur Anwendung bei der Bekämpfung und Vorbeugung cardiovaskulärer Erkrankungen.

11. Pharmazeutisches Präparat, dadurch gekennzeichnet, dass es eine Verbindung der Ansprüche 1 bis 7 oder ein Säureadditionssalz davon als Wirkstoff zusammen mit pharmazeutisch annehmbaren Träger- und Zusatzstoffen und gegebenenfalls noch eine oder mehrere andere pharmakologische Wirkstoffe enthält.

**Patentansprüche für den Vertragsstaat: AT**

1. Verfahren zur Herstellung von substituierten 3-Amino-sydnoniminen der allgemeinen Formel I

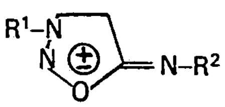

(I)

und ihre pharmakologisch annehmbaren Säureadditionssalze, worin

$R^1$ den Rest

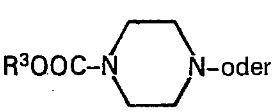

oder den Rest

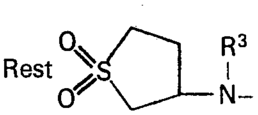

$R^2$ Wasserstoff oder den Rest $-COR^4$,

$R^3$ Methyl, Ethyl, Propyl, Isopropyl,

$R^4$ einen aliphatischen Rest mit 1 bis 4 C-Atomen, der auch durch Alkoxy mit 1 bis 3 C-Atomen substituiert sein kann, einen cycloaliphatischen Rest mit 5 bis 7 C-Atomen, einen bicycloaliphatischen Rest mit 7 bis 14 C-Atomen, einen tricycloaliphatischen Rest mit 7 bis 16 C-Atomen, einen Alkoxyrest mit 1 bis 6 C-Atomen, einen Aryloxyrest mit 6 bis 12 C-Atomen oder einen Alkoxycarbonylrest mit insgesamt 2 bis 7 C-Atomen, einen Arylrest mit 6 bis 12 C-Atomen, einen durch 1 bis 3 Halogenatome und/oder 1 bis 3 Alkylreste mit 1 bis 3 C-Atomen und/oder 1 bis 3 Alkoxyreste mit 1 bis 3 C-Atomen und/oder 1 oder 2 Nitrogruppen mono-, di- oder trisubstituierten Arylrest mit 6 bis 12 C-Atomen bedeutet, dadurch gekennzeichnet, dass eine Verbindung der allgemeinen Formel II

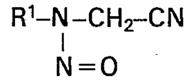

(II)

zu einer Verbindung der allgemeinen Formel Ia, die auch in Form eines Säureadditionssalzes vorliegen kann

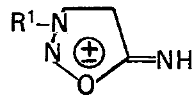

(Ia)

cyclisiert und gegebenenfalls aus dem Säureadditionssalz die freie Verbindung der Formel Ia isoliert und sie oder ein Säureadditionssalz davon mit Acylierungsmitteln, die den Rest $-COR^4$ einführen, acyliert und die erhaltene Verbindung gegebenenfalls in ein Säureadditionssalz überführt wird.

2. Verfahren nach Anspruch 1, dadurch gekennzeichnet, dass die Cyclisierung in einem Lösungs- oder Dispergiermittel bei Temperaturen von 0 bis 40 °C, vorzugsweise 0 bis 20 °C, mit Hilfe von Cyclisierungsmitteln, die in wässriger Lösung einen pH-Wert unter 3 einstellen, durchgeführt wird.

3. Verfahren nach Anspruch 1 oder 2, dadurch gekennzeichnet, dass die Ausgangsprodukte so gewählt werden, dass Verbindungen der Formel I oder pharmakologisch annehmbare Säureadditionssalze davon entstehen, wobei $R^3$ Methyl oder Ethyl, $R^4$ Alkyl mit 1 bis 4 C-Atomen, Methoxymethyl, Cycloalkyl mit 5 bis 7 C-Atomen, 2.6.6- Trimethyl-bicyclo- [3.1.1]- heptanyl, Tricyclo- $[3.3.1.1^{3,7}]$- decanyl, Methoxy, Ethoxy, Phenoxy, Ethoxycarbonyl, Phenyl, Methylphenyl, Nitrophenyl, Chlorphenyl bedeuten.

4. Verfahren nach den Ansprüchen 1 bis 3, dadurch gekennzeichnet, dass die Ausgangsverbindungen so gewählt werden, dass Verbindungen der Formel I oder pharmakologisch annehmbare Säureadditionssalze davon entstehen, wobei $R^1$ die N-Methyl-N- (tetrahydro -3-thienyl-S,S-dioxid) -amino-Gruppe bedeuten.

5. Verfahren nach den Ansprüchen 1 bis 4, dadurch gekennzeichnet, dass die Ausgangsverbindungen so gewählt werden, dass Verbindungen der Formel I oder pharmakologisch annehmbare Säureadditionssalze davon entstehen, wobei $R^2$ Wasserstoff oder die Gruppe $-COR^4$ bedeutet und $R^4$Methyl, Ethyl, Cyclohexyl, Phenyl, 4-Chlorphenyl oder 4-Nitrophenyl bedeutet.

6. Verfahren nach den Ansprüchen 1 bis 4, dadurch gekennzeichnet, dass die Ausgangsverbindungen so gewählt werden, dass die Verbindung 3- (N-Methyl-N- (tetrahydro -3- thienyl-S,S-dioxid)- amino)- sydnonimin oder ein Säureadditionssalz davon entsteht.

7. Verfahren nach den Ansprüchen 1 bis 4, dadurch gekennzeichnet, dass Ausgangsverbindungen so gewählt werden, dass die Verbindung 3-(4-Ethoxycarbonylpiperazin-1-yl)- sydnonimin oder ein Säureadditionssalz davon entsteht.

8. Verfahren nach den Ansprüchen 1 bis 4, dadurch gekennzeichnet, dass Ausgangsverbindungen so gewählt werden, dass die Verbindung 3-(N-Methyl-N- (tetrahydro- 3-thienyl-S,S-dioxid)- amino)-$N^6$-nitrobenzoyl-sydnonimin oder ein Säureadditionssalz davon entsteht.

**Revendications pour les Etats contractants: BE, CH, DE, FR, GB, IT, LI, NL, SE**

1. Amino-3-sydnone-imines substitués qui répondent à la formule générale (I):

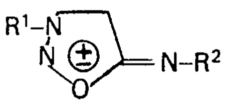 (I)

dans laquelle:

$R^1$ représente un radical répondant à l'une des formules:

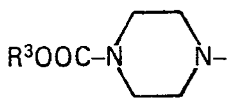 et 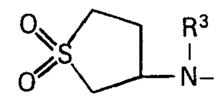

$R^2$ représente l'hydrogène ou un radical $-CO-R^4$,

$R^3$ représente un radical méthyle, éthyle, propyle ou isopropyle, et

$R^4$ représente un radical aliphatique contenant de 1 à 4 atomes de carbone, éventuellement porteur d'un alcoxy contenant de 1 à 3 atomes de carbone, un radical cycloaliphatique contenant de 5 à 7 atomes de carbone, un radical bicycloaliphatique contenant de 7 à 14 atomes de carbone, un radical tricycloaliphatique contenant de 7 à 16 atomes de carbone, un radical alcoxy contenant de 1 à 6 atomes de carbone, un radical aryloxy contenant de 6 à 12 atomes de carbone, un radical alcoxycarbonyle contenant au total de 2 à 7 atomes de carbone, un radical aryle contenant de 6 à 12 atomes de carbone, ou un radical aryle contenant de 6 à 12 atomes de carbone et portant un, deux ou trois substituants, plus précisément de 1 à 3 atomes d'halogènes et/ou de 1 à 3 radicaux alkyles en $C_1-C_3$ et/ou de 1 à 3 radicaux alcoxy en $C_1-C_3$ et/ou un ou deux radicaux nitro, ainsi que leurs sels d'addition d'acides acceptables du point de vue pharmacologique.

2. Amino-3 sydnone-imines substitués selon la revendicatin 1, caractérisés en ce que:

$R^3$ représente un radical méthyle ou éthyle,

$R^4$ représente un alkyle contenant de 1 à 4 atomes de carbone, un méthoxyméthyle, un cycloalkyle contenant de 5 à 7 atomes de carbone, un triméthyl-2,6,6 bicyclo[3.1.1] heptyle, un tricyclo [3.3.1.$1^{3,7}$] décyle, un méthoxy, un éthoxy, un phénoxy, un éthoxycarbonyle, un phényle, un méthylphényle, un nitrophényle ou un chlorophényle.

3. Amino-3 sydnone-imines substitués selon l'une des revendications 1 et 2, caractérisés en ce que $R^1$ représente un radical N-méthyl-N- (S,S-dioxyde tétrahydrothiényl-3) -amino.

4. Amino-3 sydnone-imines substitués selon l'une quelconque des revendications 1 à 3, caractérisés en ce que $R^2$ représente l'hydrogène ou un radical $-COR^4$ dont le symbole $R^4$ désigne un radical méthyle, éthyle, cyclohexyle, phényle, chlor-4 phényle ou nitro-4 phényle.

5. [N-Méthyl-N- (S,S-dioxyde tétrahydrothiényl-3)- amino]-3 sydnone-imine ou sel d'addition d'acide de celui-ci.

6. (Ethoxycarbonyl -4- piérazinyl-1) -3-sydnone-imine ou sel d'additon d'acide de celui-ci.

7. [N-Méthyl-N- (S,S-dioxyde tétrahydrothiényl-3)- amino]-3 $N^6$- (nitro- benzoyl) -sydnone-imine ou un sel d'addition d'acide de celui-ci.

8. Procédé pour préparer des composés selon l'une quelconque des revendications 1 à 7, procédé caractérisé en ce qu'on cyclise un composé de formule générale (II):

$R^1-N-CH_2-CN$ (II)
|
N=O

de manière à obtenir un composé de formule générale (Ia)

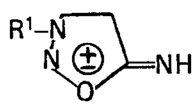 (Ia)

(composé qui peut également être sous la forme d'un sel d'addition d'acide), et éventuellement, à partir du sel d'addition d'acide, on isole le composé libre de formule (Ia), on acyle de composé ou l'un de ses sels d'addition d'acides avec un agent d'acylation capable d'introduire un radical $-COR^4$ et, le cas échéant, on convertit le composé obtenu en un sel d'addition d'acide.

9. Procédé selon la revendication 8, caractérisé en ce qu'on effectue la cyclisation au sein d'un solvant ou d'un milieu de dispersion, à des températures de 0 à 40 °C, de préférence de 0 à 20 °C, au moyen d'un agent de cyclisation qui, en solution aqueuse, donne un pH inférieur à 3.

10. Amino-3 sydnone-imines selon l'une quelconque des revendications 1 à 7, en tant que substances actives pharmacologiques pour l'application dans le traitement curatif ou le traitement préventif de maladies cardiovasculaires.

11. Composition pharmaceutique caractérisée en ce qu'elle contient un composé selon l'une quelconque des revendications 1 à 7, ou un sel d'addition d'acide d'un tel composé, en tant que substance active, associé à des excipients et à des additifs acceptables du point de vue pharmaceutique et, éventuellement, à une ou plusieurs autres substances actives douées de propriétés pharmacologiques.

**Revendications pur l'Etat contractant: AT**

1. Procédé pour préparer des amino-3-sydnone-imines substitués qui répondent à la formule générale (I):

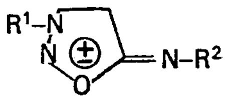

(I)

dans laquelle:

$R^1$ représente un radical répondant à l'une des formules:

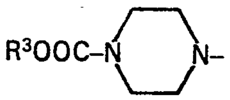 et 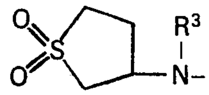

$R^2$ représente l'hydrogène ou un radical $-CO-R^4$,

$R^3$ représente un radical méthyle, éthyle, propyle ou isopropyle, et

$R^4$ représente un radical aliphatique contenant de 1 à 4 atomes de carbone, éventuellement porteur d'un alcoxy contenant de 1 à 3 atomes de carbone, un radical cycloaliphatique contenant de 5 à 7 atomes de carbone, un radical bicycloaliphatique contenant de 7 à 14 atomes de carbone, un radical tricycloaliphatique contenant de 7 à 16 atomes de carbone, un radical alcoxy contenant de 1 à 6 atomes de carbone, un radical aryloxy contenant de 6 à 12 atomes de carbone, un radical alcoxycarbonyle contenant au total de 2 à 7 atomes de carbone, un radical aryle contenant de 6 à 12 atomes de carbone, ou un radical aryle contenant de 6 à 12 atomes de carbone et portant un, deux ou trois substituants, plus précisément de 1 à 3 atomes d'halogènes et/ou de 1 à 3 radicaux alkyles en $C_1-C_3$ et/ou de 1 à 3 radicaux alcoxy en $C_1-C_3$ et/ou un ou deux radicaux nitro, ainsi que leurs sels d'addition d'acides acceptables du point de vue pharmacologique, procédé caractérisé en ce qu'on cyclise un composé de formule générale (II):

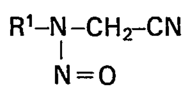

(II)

de manière à obtenir un composé de formule générale (Ia)

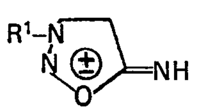

(Ia)

(composé qui peut également être sous la forme d'un sel d'addition d'acide), et éventuellement, à partir du sel d'addition d'acide, on isole le composé libre de formule (Ia), on acyle ce composé ou l'un de ses sels d'addition d'acides avec un agent d'acylation capable d'introduire un radical $-COR^4$ et, le cas échéant, on convertit le composé obtenu en un sel d'addition d'acide.

2. Procédé selon la revendication 1, caractérisé en ce qu'on effectue la cyclisation au sein d'un solvant ou d'un milieu de dispersion, à des températures de 0 à 40 °C, de préférence de 0 à 20 °C, au moyen d'un agent de cyclisation qui, en solution aqueuse, donne un pH inférieur à 3.

3. Procédé selon l'une quelconque des revendications 1 et 2, caractérisé en ce qu'on choisit les produits de départs de telle manière qu'on obtient des composés répondant à la formule I ou des sels d'addition d'acide acceptables du point de vue pharmacologique, où

$R^3$ représente un radical méthyle ou éthyle,

$R^4$ représente un alkyle contenant de 1 à 4 atomes, de carbone, un méthoxyméthyle, un cycloalkyle contenant de 5 à 7 atomes de carbone, un triméthyl-2,6,6 bicyclo [3.1.1] heptyle, un tricyclo [$3.3.1.1^{3,7}$] décyle, un méthoxy, un éthoxy, un phénoxy, un éthoxycarbonyle, un phényle, un méthylphényle, un nitrophényle ou un chlorophényle.

4. Procédé selon l'une des revendications 1 à 3 caractérisé en ce qu'on choisit les composés de départ de telle manière qu'on obtient des composés répondant à la formule I ou leurs sels d'addition d'acide acceptables du point de vue pharmacologique, ou $R^1$ représente un radical N-méthyl-N- (S,S-dioxyde tétrahydrothiényl-3) -amino.

5. Procédé selon l'une quelconque des revendications 1 à 4, caractérisé en ce qu'on choisit les composés de départ de telle manière qu'on obtient des composés répondant à la formule I ou leurs sels d'addition d'acide acceptables du point de vue pharmacologique, ou $R^2$ représente l'hydrogène ou un radical $-COR^4$ dont le symbole $R^4$ désigne un radical méthyle, ethyle, cyclohexyle, phényle, chloro-4 phényle ou nitro-4-phényle.

6. Procédé selon l'une quelconque des revendications 1 à 4, caractérisé en ce qu'on choisit les composés de départ de telle manière qu'on obtient le composé (N-méthyl-N- (S,S-dioxyde tétrahydrothiényl-3) -amino)-3-sydnone-imine ou un sel d'addition d'acide de celui-ci.

7. Procédé selon l'une quelconque des revendications 1 à 4, caractérisé en ce qu'on choisit les composés de départ de telle manière qu'on obtient le composé (ethoxycarbonyl-4 pipérazinyl-1) -3-sydnone-imine ou un sel d'addition d'acide de celui-ci.

8. Procédé selon l'une quelconque des revendications 1 à 4, caractérisé en ce qu'on choisit les composés de départ de telle manière qu'on obtient le composé (N-méthyl-N- (S,S-dioxyde tétrahydrothiényl-3) amino)-3 $N^6$-(nitro-benzoyl)-sydnone-imine ou un sel d'addition d'acide de celui-ci.

**Claims for the Contracting States BE, CH, DE, FR, GB, IT, LI, NL, SE**

1. Substituted 3-amino-sydnone-imines of the general formula I

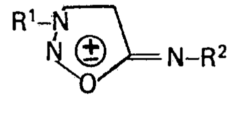

(I)

and their pharmacologically acceptable acid addition salts, wherein

$R^1$ denotes the radical

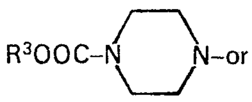 or the radical 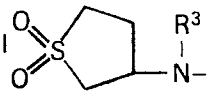

$R^2$ denotes hydrogen or the radical $-CO-R^4$,

$R^3$ denotes methyl, ethyl, propyl or isopropyl and

$R^4$ denotes an aliphatic radical with 1 to 4 C atoms, which can also be substituted by alkoxy with 1 to 3 C atoms, or denotes a cycloaliphatic radical with 5 to 7 C atoms, a bicycloaliphatic radical with 7 to 14 C atoms, a tricycloaliphatic radical with 7 to 16 C atoms, an alkoxy radical with 1 to 6 C atoms, an aryloxy radical with 6 to 12 C atoms, an alkoxycarbonyl radical with a total of 2 to 7 C atoms, an aryl radical with 6 to 12 C s toms, or an aryl radical with 6 to 12 C atoms which is monosubstituted, disubstituted or trisubstituted by 1 to 3 halogen atoms and/or 1 to 3 alkyl radicals with 1 to 3 C atoms and/or 1 to 3 alkoxy radicals with 1 to 3 C atoms and/or 1 or 2 nitro groups.

2. Substituted 3-amino-sydnone-imines according to claim 1, characterised in that

$R^3$ denotes methyl or ethyl, and

$R^4$ denotes alkyl with 1 to 4 C atoms, methoxymethyl, cycloalkyl with 5 to 7 C atoms, 2.6.6 trimethyl-bicyclo [3.1.1] -heptanyl, tricyclo-[3.3.1.$1^{3,7}$]-decanyl, methoxy, ethoxy, phenoxy, ethoxycarbonyl, phenyl, methylphenyl, nitrophenyl or chlorophenyl.

3. Substituted 3-amino-sydnone-imines according to claim 1 or 2, characterised in that $R^1$ denotes the N-Methyl-N- (tetrahydro -3- thienyl-S,S-dioxide) -amino group.

4. Substituted 3-amino-sydnone-imines according to claims 1 to 3, characterised in that $R^2$ denotes hydrogen or the group $-COR^4$ and $R^4$ denotes methyl, ethyl, cyclohexyl, phenyl, 4-chlorophenyl or 4-nitrophenyl.

5. 3-(N-methyl-N- (tetrahydro -3- thienyl S,S-dioxide) -amino sydnone-imine or an acid-addition salt thereof.

6. 3-(4-Ethoxycarbonylpiperazin -1- yl)- sydnone-imine or an acid addition salt thereof.

7. 3- (N-Methyl-N- (tetrahydro -3- thienyl-S,S-dioxide) -amino) -$N^6$- nitro-benzoyl-sydnone-imine or an acid addition salt thereof.

8. Process for the preparation of the compounds of claims 1 to 7, characterised in that a compound of the general formula II

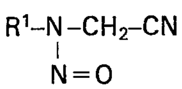 (II)

is cyclised to give a compound of the general formula Ia

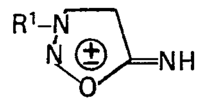 (Ia)

which can also be in the form of an acid addition salt, and, if appropriate, the free compound of the formula Ia is isolated from the acid addition salt and the free compound or an acid addition salt thereof is acylated with acylating agents which introduce the radical $-COR^4$, and, if appropriate, the resulting compound is converted into an acid addition salt.

9. Process according to claim 8, characterised in that the cyclisation is carried out in a solvent or dispersing agent at temperatures from 0 to 40 °C, preferably 0 to 20 °C, with the aid of cyclising agents which establish a pH value below 3 in aqueous solution.

10. 3-Amino-sydnone-imines of claims 1 to 7 as pharmacological active compounds, for use in combating and preventing cardiovascular illnesses.

11. Pharmaceutical product, characterised in that it contains a compound of claims 1 to 7 or an acid addition salt thereof as the active compound, together with pharmaceutically acceptable excipients and additives and, if appropriate, also one or more other pharmacologial active compounds.

**Claims for the Contracting State: AT**

1. Process for the preparation of substituted 3-amino-sydnone-imines of the general formula I

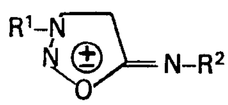 (I)

and their pharmacologically acceptable acid addition salts, wherein

$R^1$ denotes the radical

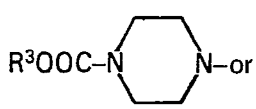 or the radical 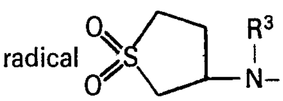

$R^2$ denotes hydrogen or the radical $-CO-R^4$,

$R^3$ denotes methyl, ethyl, propyl or isopropyl and

$R^4$ denotes an aliphatic radical with 1 to 4 C atoms, which can also be substituted by alkoxy with 1 to 3 C atoms, or denotes a cycloaliphatic radical with 5 to 7 C atoms, a bicycloaliphatic radical with 7 to 14 C atoms, a tricycloaliphatic radical with 7 to 16 C atoms, an alkoxy radical with 1 to 6 C atoms, an aryloxy radical with 6 to 12 C atoms, an alkoxycarbonyl radical with a total of 2 to 7 C atoms, an aryl radical with 6 to 12 C atoms, or an aryl radical with 6 to 12 C atoms which is monosubstituted, disubstituted or trisubstituted by 1 to 3 halogen atoms and/or 1 to 3 alkyl radicals with 1 to 3 C atoms and/or 1 to 3 alkoxy radicals with 1 to 3 C atoms and/or 1 or 2 nitro groups, characterised in that a compound of the general formula II

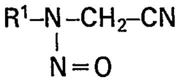
(II)

is cyclised to give a compound of the general formula Ia

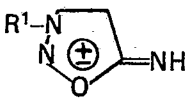
(Ia)

which can also be in the form of an acid addition salt, and, if appropriate, the free compound of the formula Ia is isolated from the acid addition salt and the free compound or an acid addition salt thereof is acylated with acylating agents which introduce the radical $-COR^4$, and, if appropriate, the resulting compound is converted into an acid addition salt.

2. Process according to claim 1, characterised in that the cyclisation is carried out in a solvent or dispersing agent at temperatures from 0 to 40 °C, preferably 0 to 20 °C, with the aid of cyclising agents which establish a pH value below 3 in aqueous solution.

3. Process according to claim 1 or 2, characterized in that the starting products are chosen thus that compounds of the formula I or pharmacologically acceptable acid addition salts thereof are obtained, wherein

$R^3$ denotes methyl or ethyl, and

$R^4$ denotes alkyl with 1 to 4 C atoms, methoxymethyl, cycloalkyl with 5 to 7 C atoms, 2.6.6 trimethyl-bicyclo [3.1.1] -heptanyl, tricyclo-[3.3.1.1$^{3,7}$] -decanyl, methoxy, ethoxy, phenoxy, ethoxycarbonyl, phenyl, methylphenyl, nitrophenyl or chlorophenyl.

4. Process according to claims 1 to 3, characterised in that the starting compounds are chosen such that compounds of formula I of pharmacologically acceptable acid addition salts thereof are obtained, wherein $R^1$ denotes the N-methyl-N-(tetrahydro -3- thienyl-S,S-dioxide) -amino group.

5. Process according to claims 1 to 4, characterised in that the starting compounds are chosen such that compounds of formula I or pharmacologically acceptable acid addition salts thereof are obtained, wherein $R^2$ denotes hydrogen or the group $-COR^4$ and $R^4$ denotes methyl, ethyl, cyclohexyl phenyl, 4-chlorophenyl or 4-nitrophenyl.

6. Process according to claims 1 to 4, characterised in that the starting compounds are chosen such that the compound 3- (N-methyl-N-(tetrahydro -3- thienyl-S,S-dioxide) -amino sydnone-imine or an acid-addition salt thereof is obtained.

7. Process according to claims 1 to 4, characterised in that the starting compounds are chosen such that the compound 3-(4-ethoxycarbonylpiperazin -1-yl) -sydnone-imine or an acid addition salt thereof is obtained.

8. Process according to claims 1 to 4, characterised in that the starting compounds are chosen such that the compound 3- (N-methyl-N-(tetrahydro -3- thienyl-S,S-dioxide) -amino) -$N^6$- nitro-benzoyl-sydnone-imine or an acid addition salt thereof is obtained.